# EUROPEAN PATENT SPECIFICATION

(11) **EP 2 668 973 B1**
(45) Date of publication and mention of the grant of the patent: **18.05.2016**
(21) Application number: 12852160.6
(22) Date of filing: 12.06.2012
(51) Int. Cl.: A61N 2/02, A61N 2/00, A61H 15/02, A61K 36/41, A61K 36/286, A61K 36/237, A61K 36/13, A61K 47/44, A61P 3/06, A61P 3/10, A61P 9/12, A61P 29/00, A61P 35/00, A61P 39/00

(54) **ELECTROMAGNETIC THERAPEUTIC APPARATUS AND SUPPORTING PHYSIOTHERAPY LIQUID THEREOF**
VORRICHTUNG FÜR ELEKTROMAGNETISCHE THERAPIEN UND UNTERSTÜTZENDE PHYSIOTHERAPIEFLÜSSIGKEIT DAFÜR
APPAREIL THÉRAPEUTIQUE ÉLECTROMAGNÉTIQUE ET SON LIQUIDE AUXILIAIRE DE PHYSIOTHÉRAPIE

(30) Priority: 22.11.2011 CN 201110371955
(43) Date of publication of application: 04.12.2013
(73) Proprietor: Shandong Chao Rui Shi Medical Technology Co., Ltd., Zibo, Shandong 255000 (CN)
(72) Inventor: WANG, Hong, Zibo Shandong 255000 (CN)
(74) Representative: Groth & Co. KB
(86) International application number: PCT/CN2012/000801
(87) International publication number: WO 2013/075391

(56) References cited:
- CN-A- 1 188 016
- CN-A- 1 190 601
- CN-A- 1 730 124
- CN-A- 1 879 594
- CN-A- 101 077 438
- CN-A- 102 091 237
- CN-Y- 2 920 028
- DE-A1- 3 938 920
- FR-A1- 2 776 932
- US-A- 5 667 469
- US-A- 5 667 469
- US-A1- 2011 224 641

## Description

### FIELD OF THE INVENTION

The present invention involves the field of medical devices and supporting liquids, and specifically, to an electromagnetic therapeutic apparatus and a supporting physiotherapy liquid thereof.

### BACKGROUND OF THE INVENTION

China is the earliest country that invented a "magnet", and Chinese ancestors discovered and used magnetic principles to various aspects of life as early as more than 2000 years ago, particularly, to the medical field, and the physiotherapy effects of a magnet have detailed disclosure in *Shennong's Herbal Classic* and *Compendium of Materia Medica.* Moreover, with the developments of modern medicine, a magnetic therapy has been listed as an important research object, as the medical frontier, in various countries.

Due to different classifications of magnetic materials, their implementation fields and applicable ranges are also different, in which the research results of a "gyromagnetic material" are the most impressive. Experiments prove that a magnetic microwave acting on a human body can change the normal motion state of the motion electric charges in the human body, which is called "field force effect". The magnetic field wave given off by the gyromagnetic material enters the human body which can achieve the purpose of diagnosing and treating diseases, and can kill cancer cells under a certain condition.

According to the report in Science and Technology Daily on June 9, 2011, the latest research result shows that magnetic fields can effectively lower the blood viscosity of human. If the blood of human is too viscous, blood pressure will increase, which will damage blood vessels and enhance the risk of heart attack. According to the report of a website http://phys.org/ on June 8 (Beijing time), physicists in Temple University in the US recently discovered that the magnetic field can be used to lower the blood viscosity of human, and the research paper was published in *Physics Review E* published recently.

Currently, the existing medical method used most widely in diluting blood is to use drugs such as aspirin, while such drugs would have side effects. Professor Rongjia Tao (transliteration) in the Temple University in the US, who once invented a method of using electric field or magnetic field to lower the oil viscosity in an engine or pipe, applied this method to controlling the blood viscosity, and discovered after testing a huge amount of blood samples that using the magnetic field could also dilute blood in the circulatory system of human.

The principle that the magnetic field dilutes blood viscosity is as follow: as red blood cells contain iron, applying the magnetic field can polarize the red blood cells so that they are linked together with short chains and in form of streamline motion. As the short chain is larger than a single blood cell, when they flow down towards the centre, their friction with blood vessel wall is reduced. Such a linking has the effect of lowering the blood viscosity and helping the blood flow more smoothly. Applying to the blood a magnetic field of 1.3T for one minute can lower the blood viscosity by 20% to 30%, and this intensity is only equivalent to that of the magnetic field of nuclear magnetic resonance imaging. When the magnetic field is removed, the blood in the blood vessel will slowly resume the initial viscous state, while this process will take several hours. *"Through selecting a suitable magnetic field intensity and a pulse time, we can control the size of the chain gathered by the red blood cells, and thus control the blood viscosity. This magnetorheological method provides an effective way which can control the blood viscosity in a selectable range",* explained Rongjia Tao, and this method is safe and can also be repeated, and can lower the blood viscosity by repeatedly applying a magnetic field, and the lowering of the viscosity will not affect the normal functions of the red blood cells.

Therefore, using the magnetic theory to cure difficult and complicated diseases, such as curing cancer and lowering blood viscosity, has been an emerging development direction of medical science, and compared with traditional operation or chemotherapy manners, its cost is low, and patients undergo greatly lowered pain, which favours the cooperation and interaction between doctors and patients and achieves prominent effects. However, the medical applications of magnetic technologies still belong to a high-end interdisciplinary branch, and currently, at home and abroad, magnetic therapy machines that have been successfully developed and have notable effects are few.

FR 2 776 932 discloses an installation for magneto-therapy with throughflow of an (aqueous) active ingredient. The installation comprises a bed to support a horizontal patient, several transversal treatment frames surrounding the couch and which may be displaced longitudinally, each frame comprising a magnetic circuit consisting of an upper magnetic plate above the bed and a lower magnetic plate below the bed. Each frame also comprises a fixed reservoir containing an active ingredient in an aqueous medium and extending from underneath the upper magnetic plate.

US 2011/0224641 discloses a magnetic conductive recipient comprising: magnetic field via magnetized water. This process provides means for drug delivery in a molecular state, control of blood circulation, control of internal and/or external cell organism functions, including MWRI (magnetic water resonance imaging).

With developments of times, applications of magnetic field in medical fields have complete theoretical basis. The applicant has been dedicated to the research and development of magnetic medicine and magnetic therapy machines for many years, and enabled multiple magnetic physiotherapy machines to be put into clinical use, thus the applicant has obtained valuable first-tier materials and accumulated experiences. The differences of the electromagnetic therapeutic apparatus developed this time from existing ones lie in that the latest research results of the magnetic medicine field and several essential physiotherapy methods of the traditional Chinese medicine are organically combined to form the overall set of apparatus, wherein mainly relying on magnetic field intervention and taking traditional Chinese medicine liquids and acupoint stimulation as supplementation, it maximizes the effects through an electromagnetic heating principle, with the multiple effects combined, and firstly obtains a magnetic physiotherapy apparatuses with traditional Chinese medicine character, home and abroad.

### SUMMARY OF THE INVENTION

The technical problem to be solved by the present invention is to provide an electromagnetic therapeutic apparatus and a supporting physiotherapy liquid thereof, which have two functions of treatment and health care.

To solve the above technical problem, the technical solution of the present invention is to provide an electromagnetic therapeutic apparatus characterizing by comprising a frame and a treatment couch, wherein more than one upper magnetic heads are mounted on the frame via a bearing and more than one lower magnetic heads are mounted inside the treatment couch, each lower magnetic head is coupled with a set of rotary driving devices respectively, so that each lower magnetic head can be driven by the rotary driving device to rotate independently; the vertical mounting positions of the lower magnetic heads correspond to those of the upper magnetic heads one by one and they are arranged opposite to each other; the rotary driving devices drive the lower magnetic heads to rotate, and the corresponding upper magnetic head is coupled to and driven by the lower magnetic head through the force of magnetic attraction; a physiotherapy cloth is placed on the treatment couch, a waterproof sealing press strip is provided at the openings of the physiotherapy cloth, and the physiotherapy cloth is connected with a physiotherapy liquid collecting tank through a circulating pump, and the physiotherapy liquid is accommodated in the physiotherapy liquid collecting tank; a liner is provided inside the physiotherapy cloth, and more than one tourmaline magnetic disc or metal block is fixed between the physiotherapy cloth and the liner.

A high-frequency induction heating coil coupled to a high-frequency power supply is mounted in the treatment couch.

The upper magnetic head and the lower magnetic head use an electromagnetic head around which a cooling device is provided, and the electromagnetic head generates in a working state an electromagnetic wave which has an induction electromotive force of 500-1000mV and a frequency of 8-20Hz; the upper magnetic heads and the lower magnetic heads are formed by joining more than two groups of electromagnetic blocks together, and gaps are kept among the respective groups of electromagnetic blocks.

The upper magnetic heads and the lower magnetic heads use a permanent magnetic head and are formed by joining more than two groups of permanent magnetic blocks together, and a gap is kept between the respective groups of permanent magnetic blocks.

The static magnetic field intensity of the upper magnetic heads and the lower magnetic heads is 0.5-0.85T (5000-8500G).

The rotation speed of the lower magnetic head is 50-600 RPM, and the distance between the upper magnetic head and the lower magnetic head is 200-400mm.

The upper magnetic head and the lower magnetic head both present a disc shape, and the ratio of the distance between centres of respective magnetic heads to the diameter of the magnetic head is 1.2:1.

The tourmaline magnetic discs or metal blocks are distributed between the physiotherapy cloth and the liner according to the acupoints of human body and cambered convex points are provided thereon.

The physiotherapy cloth has a sleeping bag-type or split-type structure.

The physiotherapy cloth having a split-type structure at least comprises a head sleeve with ear openings, a nose opening and a mouth opening provided therein, a body sleeve and limbs' sleeves, and the waterproof sealing press strips are provided at the openings of the head sleeve, the body sleeve and the limbs' sleeves, and the head sleeve, the body sleeve and the limbs' sleeves are connected with the physiotherapy liquid collecting tank, respectively.

More than one tourmaline magnetic discs or Artemisia-salt patches are placed on the upper surface of the treatment couch. Artemisia-salt is a topical composition well-known in the traditional Chinese medicine, which composition comprises one or more species of aromatic herbs in the genus *Artemisia* and one or more mineral salts.

The treatment couch is mounted with more than two latex rolling wheels which are mounted below the positions of the physiotherapy cloth corresponding to the neck bend and/or the waist bend and/or the leg bends and/or the ankle bends of human body; and the each latex rolling wheels is connected with a micro-motor which drives the latex rolling wheel to independently rotate.

Radial projections are provided on the latex rolling wheels and are embedded with Artemisia-salt particles at the tip, and a metal heating mandrel is provided inside the latex rolling wheel; after the high-frequency power supply supplies a high-frequency alternating current to the high-frequency induction heating coil, the metal block and the metal heating mandrel generate heat at the same time.

The frame comprises a column, a lifting shelf, a lifting device and an upper magnetic head mounting plate, wherein the lifting shelf is inserted into the column, and the lifting device drives the lifting shelf to perform lifting movement; the lifting shelf is connected with the upper magnetic head mounting plate, and the upper magnetic heads are mounted into the upper magnetic head mounting plate.

A heating device is mounted inside the physiotherapy liquid collecting tank and is connected with a temperature control device.

A computer control system is further incorporated, and it connects and controls the rotary driving devices, the circulating pump, the micro-motor, the lifting device and the temperature control device.

The supporting physiotherapy liquid of the electromagnetic therapeutic apparatus according to the present invention comprises a basic liquid and one or more herbal additives of traditional Chinese medicine, and the basic liquid comprises glycerol and olive oil and the mass ratio of glycerol to olive oil is 1:1 to 1:3.

The one or more herbal additives of traditional Chinese medicine are selected from the group consisting of *Angelica sinensis,* safflower, *Notopterygium, Anredera cordifolia, Taxus chinensis, Agastache rugosa* and stringy stonecrop or a combination thereof; and the mass ratio of the basic liquid to the one or more herbal additives of traditional Chinese medicine is 10:1 to 10:1.5.

The advantageous effects of the present invention are as follow:
1. When the upper magnetic heads and the lower magnetic heads use the electromagnetic head around which a cooling device is provided, the electromagnetic head generates in a working state an electromagnetic wave which has an induction electromotive force of 500-1000mV and a frequency of 8-20Hz; when the apparatus uses a permanent magnet head, through controlling the distance between the magnetic blocks, the rotation speed, the phase difference (the extent of asynchronization) between upper and lower magnetic discs and etc., a dynamic magnetic field with suitable waveform, field intensity and frequency is formed.

The dynamic magnetic field generated by one pair of magnetic heads can achieve:
(1) chest treatment: health care treatment on mammary gland and respiratory system;
(2) abdomen treatment: health care treatment on digestive system, and urinary reproductive system; and
(3) leg treatment.

The dynamic magnetic field generated by two pairs of magnetic heads can achieve:
(1) treatment on chest and abdomen at the same time: for two or more lesions; and
(2) treatment on abdomen and leg at the same time: for two or more lesions.

The dynamic magnetic field generated by three pairs of magnetic heads can achieve treatment on chest, abdomen and leg at the same time, and can improve the therapeutic efficiency for systemic diseases.

When multiple pairs of upper and lower magnetic heads which are arranged in a matrix are used, they are activated alternatively in order, so that the viscous blood in an area at the body of a patient is diffused to other tributaries, and then illness states such as blood viscosity or thrombus are alleviated.
2. For this apparatus in which a physiotherapy cloth is provided on the treatment couch and waterproof sealing press strips are provided at the openings of the physiotherapy cloth, when the patient puts on the physiotherapy cloth, by pressing the sealing press strips, the openings of the physiotherapy cloth can be sealed, which prevents the leakage of the physiotherapy liquid. The physiotherapy liquid is filled into the physiotherapy liquid collecting tank, the physiotherapy cloth is connected with the physiotherapy liquid collecting tank through a circulating pump, the physiotherapy liquid forms a uniform layered structure in the physiotherapy cloth and completely envelopes human body and can circulate, and the physiotherapy liquid can be absorbed by human body more quickly and act on the ill sites under the condition of quickening blood circulation and the metabolism of human body by magnetic therapy, so that the effects of the drug is maximized and the therapeutic effects are better.
3. The tourmaline magnetic discs or metal blocks are distributed in the physiotherapy cloth according to the acupoints of human body and are provided with cambered convex points thereon, and after the patient puts on the physiotherapy cloth, the cambered convex points can press and stimulate the acupoints of human body, thereby achieving therapeutic effects. The tourmaline magnetic disc is composited from a nano-function ceramic powder and a special thermo-induction material. When being stimulated by the temperature of human body and utilizing the effect of a transfer catalyst, the thermo-induction material immediately reacts and generates heat, and the temperature can reach 40°C, and under thermal growth, it releases far infrared and negative ions which permeate deeply into the skin, facilitating blood circulation, activating cells, facilitating the metabolism of human body, adjusting nervous system, activating endocrine, easing pain and removing coldness, which has obvious effects on arthritis and arthralgia, and it can initiatively thermally stimulate the acupoints after contacting the skin and achieve better therapeutic, rehabilitation or health care effects.
   When the metal block is used to replace the tourmaline magnetic disc, through the dynamic magnetic field provided by the upper and lower magnetic heads, the metal block can achieve magnetic induction heating, in which the temperature can reach about 43°C, and the magnetic therapeutic effects are notable. When a high temperature is needed to cooperate with the treatment, a magnetic induction heating principle (being the same with the working principle of an electromagnetic cooker) can be used to make the high-frequency power supply provide a high-frequency alternating current which is applied to a spiral high-frequency induction heating oil, thereby generating a high-frequency alternating magnetic field whose magnetic field lines act on the metal block. A strong eddy current is generated inside the metal block due to electromagnetic induction. The eddy current completes the conversion from electric energy to thermal energy when flowing against the internal resistance of the metal block, and the generated Joule heat is the thermal source for the heating, the heat amount can be controlled by adjusting the power of the high-frequency power supply, that is, the heating temperature of the metal block is adjustable and can be set accurately within the range of 40-60°C by medical care personnel according to different disease condition, so that the treatment and rehabilitation manners are more accurate and more efficient.
4. The physiotherapy cloth has a sleeping bag-type or split-type structure. The sleeping bag-type structure is suitable for the implementation of the therapeutic solution for the whole body, and it completely envelopes the body, thus makes the therapeutic effects notable. The physiotherapy cloth having a split-type structure at least comprises a head sleeve with ear openings, a nose opening and a mouth opening provided therein, a body sleeve and limbs' sleeves, and the waterproof sealing press strips are provided at the openings of the head sleeve, the body sleeve and the limbs' sleeves, and the head sleeve, the body sleeve and the limbs' sleeves are connected with the physiotherapy liquid collecting tank, respectively, and during use, it is only necessary to envelope the ill sites of the patient by the physiotherapy cloth, which is more convenient. The medical care personnel can choose a local or whole body treatment manner for the patients according to needs, which is of strong pertinence and more scientific and reasonable.
5. The treatment couch is mounted with multiple latex rolling wheels which are mounted according to a human body engineering principle at positions corresponding to the neck bend, the waist bend, the leg bends and the ankle bends of human body at the physiotherapy cloth, and effectively bear and correct the curve of the human body and help restore the correct posture of the bone and the body, and then stretch muscle and smoothly circulate the blood. Each latex rolling wheel is connected with a micro-motor and driven by it to rotate independently, and then can further strengthen under the effect of a combined magnetic field the care of physiological bends and give more massage and rehabilitation to the parts of the body which easily have pathological changes.
6. More than one tourmaline magnetic disc or Artemisia-salt patch are placed on the upper surface of the treatment couch. Radial projections are provided on the latex rolling wheels and embedded with an Artemisia-salt particles at the tip, and a metal heating mandrel is provided inside the latex rolling wheel; after the high-frequency power supply supplies a high-frequency alternating current to the high-frequency induction heating coil, the metal heating mandrel heats by itself, and heated Artemisia-salt can remove coldness and humidity, and a patient suffering from humidity and coldness can choose not to use the physiotherapy cloth and directly lies on the treatment couch, and a set of Artemisia-salt patches are prepared specially for each patient and are subjected to humidity processing each day, and the Artemisia-salt is replaced each course of treatment, and the therapeutic effects are better.
7. The basic liquid of the supporting physiotherapy liquid comprises glycerol and olive oil, the one or more herbal additives of traditional Chinese medicine can be formed as powders and then added to the basic liquid, the basic liquid surrounds the human body or ill sites, as glycerol and olive oil have strong dissolving capacity and can be easily absorbed by human body, the basic liquid can smoothly carry the traditional Chinese medicament into the circulating system of the human body. According to different disease conditions, different herbal additives of traditional Chinese medicine can be added into the basic liquid, for example, patients of diabetes, hypertension and hyperlipidaemia can use an *Anredera cordifolia* physiotherapy liquid; patients of malignant tumour use a *Taxus chinensis* physiotherapy liquid; patients of rheumatism use *Agastache rugosa* physiotherapy liquid; and a stringy stonecrop physiotherapy liquid can be used to alleviate fatigue, and etc., which is flexible and convenient and has strong pertinence. The medical care personnel can decide the medicament solutions and administration periods at different stages according to different situations of the patient, and the heating device and the temperature control device mounted in the physiotherapy liquid collecting tank can accurately control the temperature of the physiotherapy liquid and then play the effects of the medicament to the most extent.
8. The present invention is a model of perfect combination of western medicine and traditional Chinese medicine: the computer control system controls in combination the operations of the parts, and can pre-set the rotating speed of the magnetic heads, the height of the lifting shelf, the rotating speed of the latex rolling wheel and etc. according to different situations of the patients, which make the treatment more scientific and accurate. Taking advantage of western medicine magnetic principles, irradiation is applied to bodies, which has effects of cancer treatment, anti-cancer and blood viscosity dilution; meanwhile, combined with traditional Chinese medicine, the present invention speeds up the recovery process with a high metabolism, and the patient suffers no pain during the treatment and is more willing to cooperate, thus the present invention has excellent effects and is highly suitable for industrial popularization.

### BRIEF DESCRIPTION OF THE DRAWINGS

Fig. 1 is a structural schematic diagram of the present invention;
Fig. 2 is a structural schematic diagram of an A-direction of Fig. 1;
Fig. 3 is a structural schematic diagram of the upper magnetic head;
Fig. 4 is a structural schematic diagram of the B-direction of Fig. 3;
Fig. 5 is a structural schematic diagram of the metal block (or the self-heating tourmaline magnetic disc);
Fig. 6 is a structural schematic diagram of the C-C direction of Fig. 5;
Fig. 7 is a structural schematic diagram of the upper surface of the treatment couch;
Fig. 8 is a structural schematic diagram of the winding of the high-frequency induction heating coil;
Fig. 9 is a structural schematic diagram of the latex rolling wheel;
Fig. 10 is a structural schematic diagram of the D-direction of Fig. 9;
Fig. 11 is a structural schematic diagram of Embodiment 2 of the present invention;
Fig. 12 is a structural schematic diagram of the E-direction of Fig. 11;
Fig. 13 is a diagram of the distribution of the magnetic field lines between the upper magnetic heads and the lower magnetic heads;
Fig. 14 is a diagram of the distribution of the magnetic induction intensity between the upper magnetic heads and the lower magnetic heads;
Fig. 15 is a diagram showing the magnetic induction intensity at a certain point in the magnetic field between the upper magnetic heads and the lower magnetic heads changes depending on the distances between the upper magnetic heads and the lower magnetic heads; and
Fig. 16 is a diagram showing the magnetic induction intensity at a certain point in the magnetic field between the upper magnetic heads and the lower magnetic heads, changes over time.

In the drawings: 1 lifting shelf, 2 column, 3 computer control system, 4 treatment couch, 5 lower magnetic head, 6 high-frequency induction heating coil interlayer, 7 high-frequency power supply, 8 physiotherapy liquid collecting tank, 9 circulating pump, 10 latex rolling wheel, 11 tourmaline magnetic disc, 12 physiotherapy cloth, 13 upper magnetic head, 14 upper magnetic head mounting plate, 15 permanent magnetic block, 16 cambered convex point, 17 tourmaline magnetic disc, 18 high-frequency induction heating coil, 19 radial projection, 20 metal heating mandrel, 21 Artemisia-salt particle, 22 upper magnetic head, 23 metal block, 24 Artemisia-salt patch, 25 lower magnetic head.

### DETAILED DESCRIPTION OF THE PRESENT INVENTION

The present invention will be further detailed hereinafter in combination with the drawings and embodiments.

### Embodiment 1

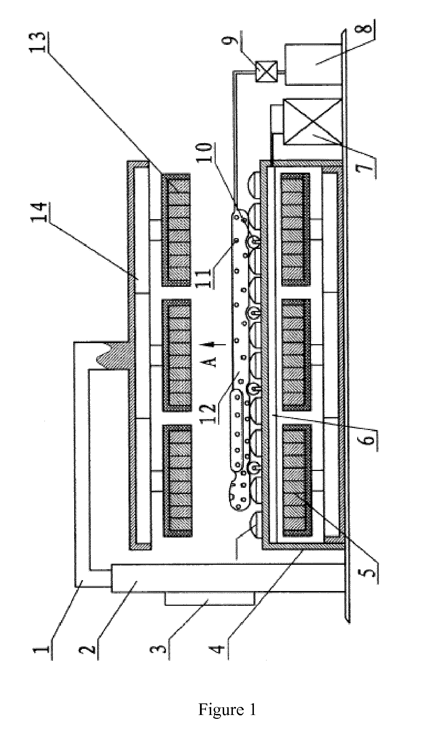

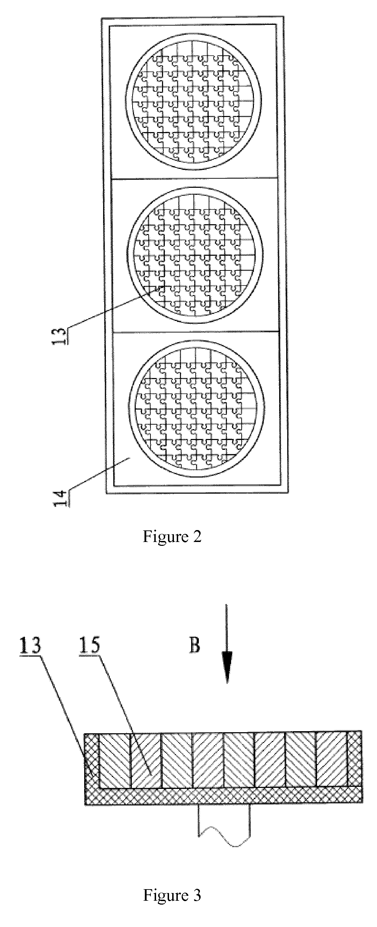

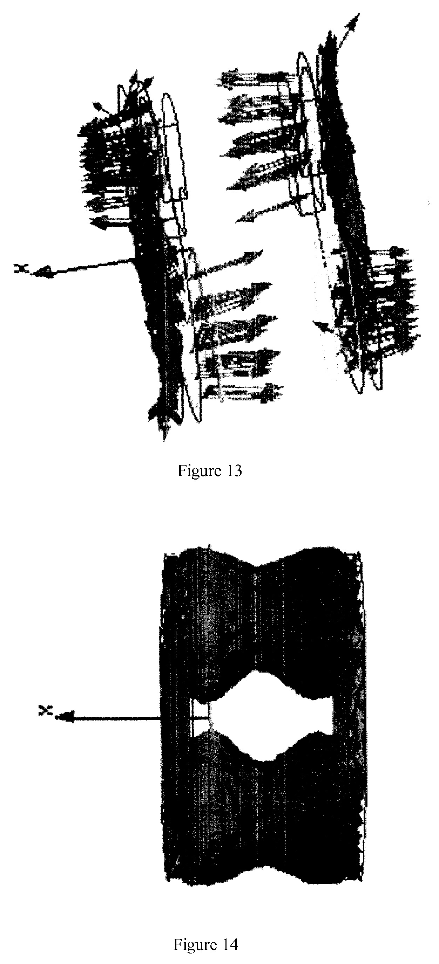

As shown in Fig. 1, it shows the electromagnetic therapeutic apparatus of the present invention, wherein the lifting shelf 1 of the frame is inserted into the column 2, and is driven by the lifting device to perform movement of going up and down; and the lifting device can be a lifting motor or cylinder, and etc. The lifting shelf 1 is connected with the upper magnetic head mounting plate 14, and three upper magnetic heads 13 are mounted on the upper magnetic head mounting plate 14 via a bearing. Below the upper magnetic heads 13 is the treatment couch 14 in which three lower magnetic heads 5 and associated supporting rotary driving devices (the rotary driving devices are micro-motors, not shown in the drawing) thereof are mounted, as shown in Fig. 2. The respective lower magnetic heads 5 can be driven by the rotary driving devices to rotate independently. The vertical positions of the lower magnetic heads 5 correspond to those of the upper magnetic heads 13 one by one and are arranged opposite to each other; the rotary driving devices drive the lower magnetic heads 5 to rotate, and the lower magnetic heads 5 couple and drive the corresponding upper magnetic heads 13 through the force of magnetic attraction. The distribution of the magnetic field lines and the distribution of the magnetic induction intensity between the upper magnetic heads and the lower magnetic heads are shown in Figs. 13 and 14.

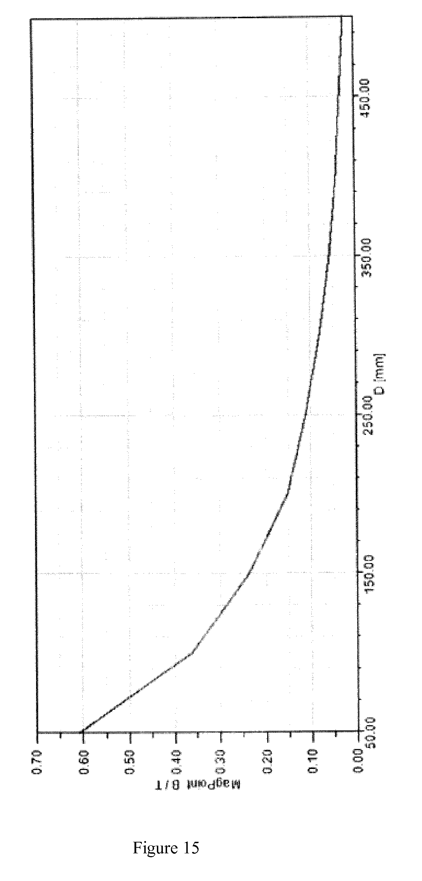

The static magnetic field intensity of the upper magnetic heads and the lower magnetic heads is 0.3T(3000G). The rotating speed of the lower magnetic heads 5 is 50 RPM, the distance between the upper magnetic heads and the lower magnetic heads is 200mm, and Fig. 15 is a diagram showing that the magnetic induction intensity at a certain point in the magnetic field between the upper magnetic heads and the lower magnetic heads changes depending on the distances between the upper magnetic heads and the lower magnetic heads, from which it is known that the longer the distance between the upper magnetic heads and the lower magnetic heads is, the weaker the magnetic field is. The upper magnetic heads and the lower magnetic heads present a disc shape, and the ratio of the distance between centres of respective magnetic heads to the diameter of the magnetic head is 1.2:1.

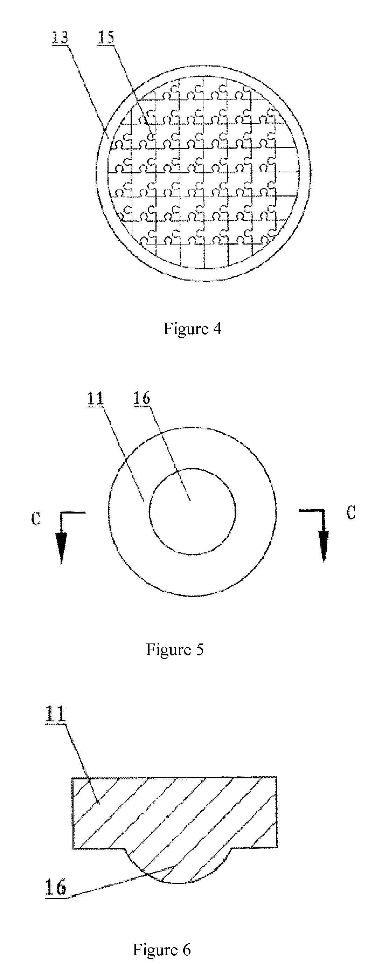

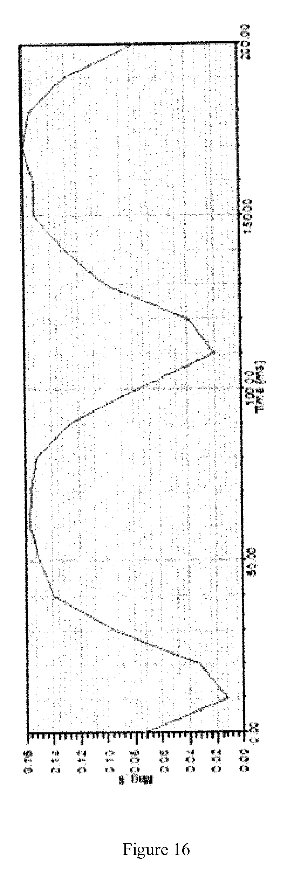

As shown in Figs. 3 and 4, the upper magnetic heads and the lower magnetic heads use a permanent magnetic head, and are formed by joining more than two groups of permanent magnetic blocks 15 together. Between the individual groups of permanent magnetic blocks 15, a gap is kept. When the magnetic heads rotate, the magnetic fields of the upper magnetic heads and the lower magnetic heads change alternatively due to a phase difference (that is, the asynchronization of the upper magnetic heads and the lower magnetic heads during rotation), thereby generating a pulsing effect of therapeutic functions. As shown in Fig. 16, the magnetic induction intensity at a certain point in the magnetic field between the upper magnetic heads and the lower magnetic heads changes over time and presents a pulsing fluctuation, and thus intervenes the magnetic field of human body and fulfils treatment.

The arrangement of the magnetic blocks is calculated according to the waveform, the amplitude and the frequency of a desired dynamic magnetic field, and the magnetic structure arranged according to calculated data is subjected to practical test and compared with the standard waveform, and mounted if found to be errorless after check. Selected dynamic magnetic field parameters (waveform, induction electromotive force, frequency, duration of each treatment, and treatment period) will enhance the biological current of the human body, improve the oxygen carrying ability, running speed and range of the red blood cells, then improve the metabolic ability of the human body, and then treat and rehabilitate multiple chronic diseases including malignant tumour.

Three pairs of upper magnetic heads and lower magnetic heads respectively correspondingly irradiate the chest, abdomen and legs of the human body, and different upper magnetic heads and lower magnetic heads can be rotated for different ill sites, to achieve the effects of gyromagnetic curing and restoring health.

A physiotherapy cloth 12 is placed on the treatment couch 4 and has a sleeping bag-type structure. A waterproof zipper is provided at one side of the sleeping bag, to make the patient convenient to put on it. The head part of the physiotherapy cloth 12 is provided with openings for ears, nose and throat where waterproof sealing press strips are provided, and the patient compacts the waterproof sealing press strips after putting on the physiotherapy cloth 12 to prevent the leakage of the physiotherapy liquid.

As shown in Figs. 1, 5 and 6, a liner is provided inside the physiotherapy cloth 12, and the tourmaline magnetic discs 11 are fixed between the physiotherapy cloth 12 and the liner, and distributed in the physiotherapy cloth 12 according to the acupoints of human body and provided with cambered convex points 16 thereon. The tourmaline magnetic disc 11, after contacting skin, can generate heat by itself in the case of meeting water and then stimulate acupoints, and then achieve better therapeutic or rehabilitation and health care effect.

The physiotherapy cloth 12 is connected through the circulating pump 9, the physiotherapy liquid is filled into the physiotherapy liquid collecting tank 8 and can form a uniform layered structure in the physiotherapy cloth 12 under the effect of the circulating pump 9, and completely envelopes human body and can circulate, and the physiotherapy liquid can be absorbed by human body more quickly and act on the ill sites under the condition of quickening blood circulation and the metabolism of human body by magnetic therapy. A heating device is mounted in the physiotherapy liquid collecting tank 8 and is connected with a temperature control device, and can heat in advance the physiotherapy liquid to a suitable temperature which is usually within the range of 40-60°C, and the physiotherapy liquid is injected into the physiotherapy cloth 12 through the circulating pump 9, which can improve the effects of the physiotherapy liquid more obviously.

The basic formula of the physiotherapy liquid comprises a basic liquid and one or more herbal additives of traditional Chinese medicine, and the basic liquid comprises glycerol and olive oil and the mass ratio of glycerol to olive oil is 1:1.

When used to patients with different diseases, different medicines can be added according to different illness reasons to assist treatment. The one or more herbal additives of traditional Chinese medicine are selected from the group consisting of *Angelica sinensis,* safflower, *Notopterygium, Anredera cordifolia, Taxus chinensis, Agastache rugosa* and stringy stonecrop or a combination thereof; and the mass ratio of the basic liquid to the one or more herbal additives of traditional Chinese medicine is about 10:1.

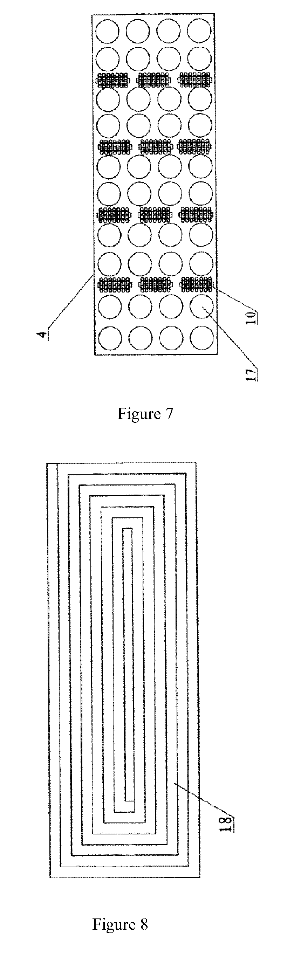

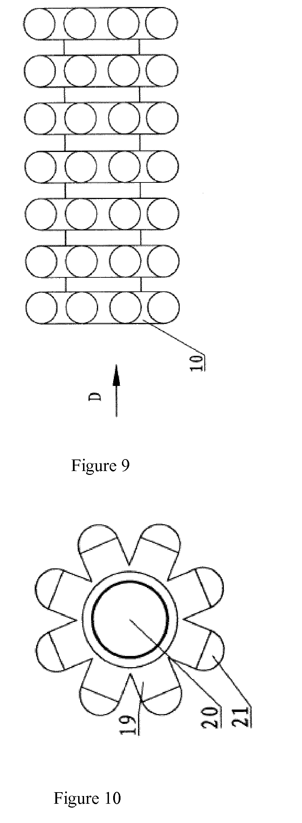

As shown in Fig. 7, the tourmaline magnetic disc is placed on the upper surface of the treatment couch 4, and the treatment couch 4 is mounted with more than two latex rolling wheels 10 which are mounted below the positions of the physiotherapy cloth 12 corresponding to the neck bend, the waist bend, the leg bends and the ankle bends of human body; each latex rolling wheel 10 is connected with a micro-motor and driven by it to rotate independently, thereby achieving a massage effect. As shown in Figs. 9 and 10, radial projections 19 are provided on the latex rolling wheels 10 and are embedded with Artemisia-salt particles 21 at the tip, and a metal heating mandrel 20 is provided inside the latex rolling wheel 10. The high-frequency induction heating coil interlayer 6 is provided inside the treatment couch 4, and the high-frequency induction heating coil 18 is spirally wound and arranged inside the interlayer, as shown in Fig. 8. After the high-frequency power supply 7 supplies a high-frequency alternating current, the metal heating mandrel 20 generates heat by itself under the effect of electromagnetic induction, and heated Artemisia-salt can remove coldness and humidity, and a patient suffering from humidity and coldness can choose not to use the physiotherapy cloth 12 and directly lies on the treatment couch 4, and a set of mattress is specially prepared for each patient and is subjected to humidity removing each day, the Artemisia-salt is replaced in each course of treatment, and the therapeutic effects are better.

During the use of the present invention, the computer control system 3 is connected with and controls the rotary driving devices, the circulating pump 9, the micro-motors, the lifting device and the temperature control device, and can pre-set the rotating speed of the magnetic heads, the height of the lifting shelf 1, the rotating speed of the latex rolling wheel 10, the temperature of the heated physiotherapy liquid and etc. according to different situations of the patients, which make the treatment more scientific and accurate. The additives of the physiotherapy liquid inside the physiotherapy cloth 12 can be selected according to needs of the patients, or the patients can also directly lie on the treatment couch 4. Using the magnetic rotation effect of the upper magnetic heads and the lower magnetic heads makes the treatment flexible and it can be operated conveniently and easily without pains.

### Embodiment 2

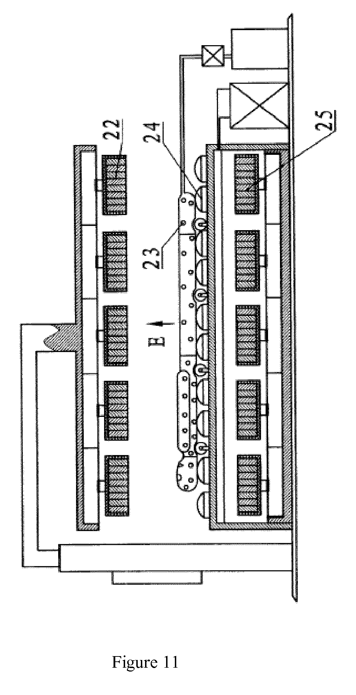

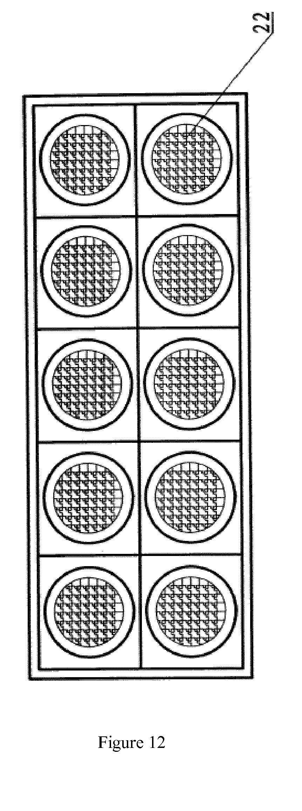

As shown in Figs. 11 and 12, the upper magnetic heads and the lower magnetic heads use the electromagnetic heads in the number of 10 around which a cooling device is provided, and the electromagnetic heads generate in a working state an electromagnetic wave which has an induction electromotive force of 500mV and a frequency of 8Hz. The positions of the lower magnetic heads 25 correspond to those of the upper magnetic heads 22 respectively and are arranged opposite to each other; the rotary driving devices drive the lower magnetic heads 25 to rotate, and the corresponding upper magnetic heads 22 are driven by the lower magnetic heads 25 through the force of magnetic attraction. The static magnetic field intensity of the upper magnetic heads and the lower magnetic heads is 0.85T(8500G). The rotating speed of the lower magnetic heads 25 is 600 RPM. This structure not only generates good gyromagnetic therapeutic effect, and activates the upper magnetic heads and lower magnetic heads at different sites alternatively, so that viscous blood at an area of the body of a patient is diffused to individual tributaries, and then the illness state is alleviated.

The physiotherapy cloth 12 is of the split-type structure, and comprises a head sleeve with ear openings, a nose opening and a mouth opening provided therein, a body sleeve and limbs' sleeves, and the waterproof sealing press strips are provided at the openings of the head sleeve, the body sleeve and the limbs' sleeves, and the head sleeve, the body sleeve and the limbs' sleeves are connected with the physiotherapy liquid collecting tank 8, respectively. The medical care personnel can choose a local treatment manner or a whole body treatment manner for the patients according to needs, which is of strong pertinence and more scientific and reasonable.

The tourmaline magnetic discs placed on the upper surface of the treatment couch 4 are replaced with the Artemisia-salt patches 24, and the tourmaline magnetic discs 11 fixed in the physiotherapy cloth 12 are replaced with the metal blocks 23. The metal blocks 23, having the same structure with the tourmaline magnetic discs 11, are also distributed in the physiotherapy cloth 12 according to acupoints of the human body and also provided with the cambered convex points 16. The high-frequency induction heating coil interlayer 6 is provided inside the treatment couch 4, and the high-frequency induction heating coil 18 is spirally wound and arranged inside the interlayer, as shown in Fig. 8. After the high-frequency power supply 7 supplies a high-frequency alternating current, an electromagnetic induction heating principle (being the same with the working principle of an electromagnetic cooker) is used for the high-frequency power supply 7 to apply the high-frequency alternating current to the spiral high-frequency induction heating coil 18, thereby generating a high-frequency alternating magnetic field whose magnetic field lines act on the metal blocks 23. A strong eddy current is generated inside the metal block 23 due to electromagnetic induction. The eddy current completes the conversion from electric energy to thermal energy when flowing against the internal resistance of the metal blocks 23, and the generated Joule heat is the thermal source for the heating, the heat amount can be controlled by adjusting the power of the high-frequency power supply 7, that is, the heating temperature of the metal block 23 is adjustable and can be set accurately within the range of 40-60°C according to different illness states, so that the treatment and rehabilitation manners are more accurate and more efficient.

The mass ratio of glycerol to olive oil is 1:2, and the mass ratio of the basic liquid to the one or more herbal additives of traditional Chinese medicine is 10:1.2.

The other structures and using methods of this embodiment are the same with Embodiment 1 and then are omitted here.

### Embodiment 3

As shown in Figs. 11 and 12, the upper magnetic heads and the lower magnetic heads use the electromagnetic heads in the number of 10, and the electromagnetic heads generate in a working state an electromagnetic wave which has an induction electromotive force of 1000mV and a frequency of 20Hz. The static magnetic field intensity of the upper magnetic heads and the lower magnetic heads is 0.85T(8500G). The rotating speed of the lower magnetic heads 25 is 430 RPM. The distance between the upper magnetic heads and the lower magnetic heads is 350mm.

The mass ratio of glycerol to olive oil is 1:3, and the mass ratio of the basic liquid to the one or more herbal additives of traditional Chinese medicine is 10:1.5.

The other structures and using methods of this embodiment are the same with Embodiment 1 and then are omitted here.

### Embodiment 4

The upper magnetic heads and the lower magnetic heads use the electromagnetic heads in the number of 10, and the electromagnetic heads generate in a working state an electromagnetic wave which has an induction electromotive force of 2000mV and a frequency of 30Hz. The static magnetic field intensity of the upper magnetic heads and the lower magnetic heads is 0.65T(6500G). The rotating speed of the lower magnetic heads 25 is 560 RPM. The distance between the upper magnetic heads and the lower magnetic heads is 400mm.

The other structures and using methods of this embodiment are the same with embodiment 1 and then are omitted here.

It is needed to be indicated that the above embodiments are merely preferred ones of the present invention and the accompanying drawings of the description only serve to explain the embodiments. For one skilled in the art, under the precondition of complying with the working principles of the present invention, any equivalent or similar substitutions shall all be covered in the scope of protection of the present invention.

## Claims

1. An electromagnetic therapeutic apparatus comprising a frame, a treatment couch (4), a physiotherapy liquid collecting tank (8) and a physiotherapy liquid;
**characterized in that** the apparatus further comprises more than one upper magnetic heads (13; 22) rotatably mounted on the frame via a bearing and more than one lower magnetic heads (5; 25) mounted inside the treatment couch (4), wherein each lower magnetic head (5; 25) is coupled with a set of rotary driving devices respectively, so that each lower magnetic head (5; 25) can be driven by a rotary driving device to rotate independently;
wherein the vertical mounting positions of the lower magnetic heads (5; 25) correspond to those of the upper magnetic heads (13; 22) one by one and are arranged opposite to each other;
wherein the rotary driving devices are adapted to drive the lower magnetic heads (5; 25) to rotate, and each upper magnetic head (13; 22) is coupled to and adapted to be driven to rotate by the corresponding lower magnetic head (5; 25) through the force of magnetic attraction;
a physiotherapy cloth (12) adapted to envelope at least a part of a human body and placed on the treatment couch (4), wherein waterproof sealing press strips are provided at the openings of the physiotherapy cloth (12), and the physiotherapy cloth (12) is connected with the physiotherapy liquid collecting tank (8) through a circulating pump (9), the physiotherapy liquid collecting tank (8) containing the physiotherapy liquid; and
wherein a liner is provided inside the physiotherapy cloth (12), and more than one tourmaline magnetic discs (11; 17) or metal blocks (23) are fixed between the physiotherapy cloth (12) and the liner.

2. The electromagnetic therapeutic apparatus according to claim 1, wherein a high-frequency induction heating coil (18) coupled to a high-frequency power supply (7) is mounted in the treatment couch (4).

3. The electromagnetic therapeutic apparatus according to claim 2, wherein the upper magnetic heads (13; 22) and the lower magnetic heads (5; 25) are provided with an electromagnetic head around which a cooling device is provided, and the electromagnetic head generates in a working state an electromagnetic wave which has an induction electromotive force of 500-1000mV and a frequency of 8-20Hz;
the upper magnetic heads (13; 22) and the lower magnetic heads (5; 25) are formed by joining more than two groups of electromagnetic blocks (15) together, and a gap is kept between the respective groups of electromagnetic blocks (15).

4. The electromagnetic therapeutic apparatus according to claim 2, wherein the upper magnetic heads (13; 22) and the lower magnetic heads (5; 25) are provided with a permanent magnetic head and are formed by joining more than two groups of permanent magnetic blocks (15) together, and a gap is kept between the respective groups of permanent magnetic blocks (15).

5. The electromagnetic therapeutic apparatus according to claim 3 or 4, wherein the static magnetic field intensity between the upper magnetic heads (13; 22) and the lower magnetic heads (5; 25) is 0.5-0.85T, (5000-8500 G) the rotation speed of the lower magnetic heads (5; 25) is 50-600RPM, and the distance between the upper magnetic heads (13; 22) and the lower magnetic heads (5; 25) is 200-400mm, wherein the upper magnetic heads (13; 22) and the lower magnetic heads (5; 25) both present a disc shape, and the ratio of the distance between centres of respective magnetic heads to the diameter of the magnetic head is 1.2:1.

6. The electromagnetic therapeutic apparatus according to claim 5, wherein the tourmaline magnetic discs (11; 17) or metal blocks (23) are distributed between the physiotherapy cloth (12) and the liner according to the acupoints of a human body and are provided with cambered convex points (16) thereon.

7. The electromagnetic therapeutic apparatus according to claim 6, wherein the physiotherapy cloth (12) is of a sleeping bag-type or split-type structure, the physiotherapy cloth (12) of the split-type structure at least comprising a head sleeve with ear openings, a nose opening and a mouth opening provided therein, a body sleeve and limbs' sleeves, wherein the waterproof sealing press strips are provided at the openings of the head sleeve, the body sleeve and the limbs' sleeves, and the head sleeve, and wherein the body sleeve and the limbs' sleeves are connected with the physiotherapy liquid collecting tank (8), respectively.

8. The electromagnetic therapeutic apparatus according to claim 7, wherein more than one tourmaline magnetic disc (11; 17) or Artemisia-salt patch (24) are placed on the upper surface of the treatment couch (4).

9. The electromagnetic therapeutic apparatus according to claim 8, wherein the treatment couch (4) is mounted with two or more latex rolling wheels (10) which are mounted below the positions of the physiotherapy cloth (12) corresponding to the neck bend and/or the waist bend and/or the leg bends and/or the ankle bends of a human body; and each latex rolling wheel (10) is connected with a micro-motor which drives the latex rolling wheel (10) to independently rotate.

10. The electromagnetic therapeutic apparatus according to claim 9, wherein radial projections (19) are provided on the latex rolling wheels (10) and embedded with Artemisia-salt particles (21) at the tip, and a metal heating mandrel (20) is provided inside the latex rolling wheel (10);
wherein the high-frequency power supply (7) is adapted to supply a high-frequency alternating current to the high-frequency induction heating coil (18), such that the metal block (23) and the metal heating mandrel (20) generate heat at the same time.

11. The electromagnetic therapeutic apparatus according to claim 10, wherein the frame comprises a column (2), a lifting shelf (1), a lifting device and an upper magnetic head mounting plate (14), wherein the lifting shelf (1) is inserted into the column (2), and the lifting device is adapted to drive the lifting shelf (1) to perform movement of going up and down; wherein the lifting shelf (1) is connected with the upper magnetic head mounting plate (14), and the upper magnetic heads (13; 22) are mounted in the upper magnetic head mounting plate (14).

12. The electromagnetic therapeutic apparatus according to claim 11, wherein a heating device is mounted inside the physiotherapy liquid collecting tank (8) and is connected with a temperature control device.

13. The electromagnetic therapeutic apparatus according to claim 12, wherein it further comprises a computer control system (3) which is adapted to connect and control the rotary driving devices, the circulating pump (9), the micro-motor, the lifting device and the temperature control device.

14. The electromagnetic therapeutic apparatus according to any preceding claim, wherein the physiotherapy liquid comprises a basic liquid and one or more herbal additives of traditional Chinese medicine, wherein the basic liquid comprises glycerol and olive oil, and the mass ratio of the glycerol to the olive oil is 1:1 to 1:3, wherein the one or more herbal additives of traditional Chinese medicine are selected from the group consisting of *Angelica sinensis,* safflower, *Notopterygium, Anredera cordifolia, Taxus chinensis, Agastache rugosa* and stringy stonecrop or a combination thereof; and the mass ratio of the basic liquid to the one or more herbal additives of traditional Chinese medicine is 10:1 to 10:1.5.

## Patentansprüche

1. Elektromagnetische Therapievorrichtung, die einen Rahmen, eine Behandlungsliege (4), einen Physiotherapieflüssigkeit sammelnden Behälter (8) und eine Physiotherapieflüssigkeit umfasst;
**dadurch gekennzeichnet, dass** die Vorrichtung ferner mehr als einen oberen Magnetkopf (13; 22), der auf dem Rahmen über ein Lager drehbar angebracht ist, und mehr als einen unteren Magnetkopf (5; 25), der innerhalb der Behandlungsliege (4) angebracht ist, umfasst, wobei jeder untere Magnetkopf (5; 25) jeweils an eine Gruppe von Drehantriebsvorrichtungen gekoppelt ist, so dass jeder untere Magnetkopf (5; 25) durch eine Drehantriebsvorrichtung so angetrieben werden kann, dass er sich unabhängig dreht;
wobei die vertikalen Befestigungspositionen der unteren Magnetköpfe (5; 25) denen der oberen Magnetköpfe (13; 22) eins zu eins entsprechen und einander gegenüberliegend angeordnet sind;
wobei die Drehantriebsvorrichtungen ausgelegt sind, die unteren Magnetköpfe (5; 25) zu drehen, und jeder obere Magnetkopf (13; 22) angekoppelt ist und ausgelegt ist, so angetrieben zu werden, dass er sich durch den entsprechenden unteren Magnetkopf (5; 25) durch die magnetische Anziehungskraft dreht;
ein Physiotherapietuch (12), das ausgelegt ist, mindestens einen Teil eines menschlichen Körpers zu umhüllen, und das auf der Behandlungsliege (4) positioniert ist, wobei wasserdichte Verschlussdruckstreifen an den Öffnungen des Physiotherapietuchs (12) vorgesehen sind und das Physiotherapietuch (12) mit dem physiotherapieflüssigkeitssammelnden Behälter (8) durch eine Zirkulationspumpe (9) verbunden ist, wobei der Physiotherapieflüssigkeit sammelnde Behälter (8) die Physiotherapieflüssigkeit enthält; und
wobei eine Auskleidung innerhalb des Physiotherapietuchs (12) vorgesehen ist und mehr als eine Turmalin-Magnetscheibe (11; 17) oder ein Turmalin-Metallblock (23) zwischen dem Physiotherapietuch (12) und der Auskleidung vorgesehen sind.

2. Elektromagnetische Therapievorrichtung nach Anspruch 1, wobei eine Hochfrequenzinduktionsheizspule (18), die an eine Hochfrequenzstromversorgung (7) gekoppelt ist, an der Behandlungsliege (4) befestigt ist.

3. Elektromagnetische Therapievorrichtung nach Anspruch 2, wobei die oberen Magnetköpfe (13; 22) und die unteren Magnetköpfe (5; 25) mit einem elektromagnetischen Kopf ausgestattet sind, um den eine Kühlvorrichtung vorgesehen ist, und der elektromagnetische Kopf in einem Arbeitszustand eine elektromagnetische Welle erzeugt, die eine induktive Urspannung von 500-1000 mV und eine Frequenz von 8-20 Hz aufweist;
die oberen Magnetköpfe (13; 22) und die unteren Magnetköpfe (5; 25) durch Verbinden von mehr als zwei Gruppen von elektromagnetischen Blöcken (15) miteinander gebildet sind und ein Abstand zwischen den jeweiligen Gruppen der elektromagnetischen Blöcke (15) beibehalten wird.

4. Elektromagnetische Therapievorrichtung nach Anspruch 2, wobei die oberen Magnetköpfe (13; 22) und die unteren Magnetköpfe (5; 25) mit einem Permanentmagnetkopf ausgestattet sind und durch Verbinden von mehr als zwei Gruppen von Permanentmagnetblöcken (15) miteinander gebildet sind und ein Abstand zwischen den jeweiligen Gruppen von Permanentmagnetblöcken (15) beibehalten wird.

5. Elektromagnetische Therapievorrichtung nach Anspruch 3 oder 4, wobei die statische Magnetfeldstärke zwischen den oberen Magnetköpfen (13; 22) und den unteren Magnetköpfen (5; 25) 0,5-0,85 T (5000-8500 G) beträgt, die Drehzahl der unteren Magnetköpfe (5; 25) 50-600 min⁻¹ beträgt und der Abstand zwischen den oberen Magnetköpfen (13; 22) und den unteren Magnetköpfen (5; 25) 200-400 mm beträgt, wobei die oberen Magnetköpfe (13; 22) und die unteren Magnetköpfe (5; 25) jeweils eine Scheibenform aufweisen und das Verhältnis des Abstands zwischen den Mittelpunkten der jeweiligen Magnetköpfe zu dem Durchmesser des Magnetkopfes 1,2:1 beträgt.

6. Elektromagnetische Therapievorrichtung nach Anspruch 5, wobei die Turmalin-Magnetscheiben (11; 17) oder -Metallblöcke (23) zwischen dem Physiotherapietuch (12) und der Auskleidung gemäß den Akupunkten eines menschlichen Körpers verteilt sind und mit gekrümmten Konvexpunkten (16) darauf vorgesehen sind.

7. Elektromagnetische Therapievorrichtung nach Anspruch 6, wobei das Physiotherapietuch (12) von einem Schlafsacktyp oder einer spaltartigen Struktur ist, wobei das Physiotherapietuch (12) der spaltartigen Struktur mindestens eine Kopfhülle mit Ohröffnungen, einer Nasenöffnung und einer Mundöffnung, die darin vorgesehenen sind, eine Körperhülle und eine Gliedmaßenhülle umfasst, wobei die wasserdichten Verschlussdruckstreifen an den Öffnungen der Kopfhülle, der Körperhülle und den Gliedmaßenhüllen vorgesehen sind und der Kopfhülle und wobei die die Körperhülle und die Gliedmaßenhüllen jeweils mit dem Physiotherapieflüssigkeit sammelnden Behälter (8) verbunden sind.

8. Elektromagnetische Therapievorrichtung nach Anspruch 7, wobei mehr als eine Turmalin-Magnetscheibe (11; 17) oder mehr als ein Beifußsalzkissen (24) auf der oberen Fläche der Behandlungsliege (4) positioniert sind.

9. Elektromagnetische Therapievorrichtung nach Anspruch 8, wobei die Behandlungsliege (4) mit zwei oder mehr Latexrollrädern (10), die unter den Positionen des Physiotherapietuchs (12), die der Nackenbiegung, und/oder der Taillenbiegung und/oder der Beinbiegung und/oder der Fußgelenkbiegung eines menschlichen Körpers entsprechen, angebracht sind, eingerichtet ist; und jedes Latexrollrad (10) mit einem Mikromotor verbunden ist, der das Latexrollrad (10) so antreibt, dass es sich unabhängig dreht.

10. Elektromagnetische Therapievorrichtung nach Anspruch 9, wobei radiale Vorsprünge (19) auf den Latexrollrädern (10) vorgesehen sind und an der Spitze in Beifußsalzteilchen (21) eingebettet sind und eine Metallheizspindel (20) innerhalb des Latexrollrads (10) vorgesehen ist;
wobei die Hochfrequenzstromversorgung (7) ausgelegt ist, einen Hochfrequenzwechselstrom an die Hochfrequenzinduktionsheizspule (18) derart zu liefern, dass der Metallblock (23) und die Metallheizspindel (20) gleichzeitig Wärme erzeugen.

11. Elektromagnetische Therapievorrichtung nach Anspruch 10, wobei der Rahmen eine Stütze (2), ein Heberegal (1), eine Hebevorrichtung und eine obere Magnetkopfbefestigungsplatte (14) umfasst, wobei das Heberegal (1) in die Stütze (2) eingefügt ist und die Hebevorrichtung ausgelegt ist, das Heberegal (1) anzutreiben, eine Bewegung des Hoch- und Runterfahrens auszuführen; wobei das Heberegal (1) mit der oberen Magnetkopfbefestigungsplatte (14) verbunden ist und die oberen Magnetköpfe (13; 22) in der oberen Magnetkopfbefestigungsplatte (14) angebracht sind.

12. Elektromagnetische Therapievorrichtung nach Anspruch 11, wobei eine Heizvorrichtung innerhalb des Physiotherapieflüssigkeit sammelnden Behälters (8) angebracht ist und mit einer Temperatursteuervorrichtung verbunden ist.

13. Elektromagnetische Therapievorrichtung nach Anspruch 12, wobei sie ferner ein Computersteuersystem (3) umfasst, das ausgelegt ist, die Drehantriebsvorrichtungen, die Zirkulationspumpe (9), den Mikromotor, die Hebevorrichtung und die Temperatursteuervorrichtung zu verbinden und zu steuern.

14. Elektromagnetische Therapievorrichtung nach einem vorhergehenden Anspruch, wobei die Physiotherapieflüssigkeit eine Basisflüssigkeit und einen oder mehrere pflanzliche Zusätze der traditionellen chinesischen Medizin umfasst, wobei die Basisflüssigkeit Glycerin und Olivenöl umfasst und das Massenverhältnis des Glycerins zu Olivenöl 1:1 bis 1:3 beträgt, wobei der eine oder die mehreren pflanzlichen Zusätze der traditionellen chinesischen Medizin aus der Gruppe ausgewählt sind, die aus chinesischem Engelwurz, Saflor, Notopterygium, Madeirawein, chinesischer Eibe, koreanischer Minze, scharfem Mauerpfeffer und Kombinationen davon besteht; und das Massenverhältnis der Basisflüssigkeit zu dem einen oder den mehreren pflanzlichen Zusätzen der traditionellen chinesischen Medizin 10:1 bis 10:1,5 beträgt.

## Revendications

1. Appareil thérapeutique électromagnétique comprenant un cadre, une couche de traitement (4), un réservoir de collecte de liquide de physiothérapie (8) et un liquide de physiothérapie;
**caractérisé en ce que** l'appareil comprend en outre plusieurs têtes magnétiques supérieures (13; 22) montées de façon rotative sur le cadre par l'intermédiaire d'un palier, et plusieurs têtes magnétiques inférieures (5; 25) montées à l'intérieur de la couche de traitement (4), dans lequel chaque tête magnétique inférieure (5; 25) est couplée à un ensemble de dispositifs d'entraînement rotatifs respectivement, de telle sorte que chaque tête magnétique inférieure (5; 25) puisse être entraînée par un dispositif d'entraînement rotatif afin de tourner de façon indépendante;
dans lequel les positions de montage verticales des têtes magnétiques inférieures (5; 25) correspondent à celles des têtes magnétiques supérieures (13; 22) une par une et sont agencées les unes en face des autres;
dans lequel les dispositifs d'entraînement rotatifs sont aptes à entraîner les têtes magnétiques inférieures (5; 25) à tourner, et chaque tête magnétique supérieure (13; 22) est couplée à et est apte à être entraînée à tourner par la tête magnétique inférieure correspondante (5; 25) par l'entremise de la force d'attraction magnétique;
une toile de physiothérapie (12) apte à envelopper au moins une partie d'un corps humain et placée sur la couche de traitement (4), dans lequel des bandes de pression de scellage étanche à l'eau sont prévues aux ouvertures de la toile de physiothérapie (12), et la toile de physiothérapie (12) est connectée au réservoir de collecte de liquide de physiothérapie (8) à travers une pompe de circulation (9), le réservoir de collecte de liquide de physiothérapie (8) contenant le liquide de physiothérapie; et
dans lequel une doublure est prévue à l'intérieur de la toile de physiothérapie (12), et plusieurs disques magnétiques de tourmaline (11; 17) ou blocs métalliques (23) sont fixés entre la toile de physiothérapie (12) et la doublure.

2. Appareil thérapeutique électromagnétique selon la revendication 1, dans lequel une bobine de chauffage par induction à haute fréquence (18) couplée à une alimentation électrique à haute fréquence (7) est montée dans la couche de traitement (4).

3. Appareil thérapeutique électromagnétique selon la revendication 2, dans lequel les têtes magnétiques supérieures (13; 22) et les têtes magnétiques inférieures (5; 25) sont pourvues d'une tête électromagnétique autour de laquelle un dispositif de refroidissement est prévu, et la tête électromagnétique génère dans un état de travail une onde électromagnétique qui présente une force électromotrice d'induction de 500 mV à 1000 mV et une fréquence de 8 Hz à 20 Hz;
les têtes magnétiques supérieures (13; 22) et les têtes magnétiques inférieures (5; 25) sont formées en joignant plus de deux groupes de blocs électromagnétiques (15) les uns aux autres, et un espace est maintenu entre les groupes respectifs de blocs électromagnétiques (15).

4. Appareil thérapeutique électromagnétique selon la revendication 2, dans lequel les têtes magnétiques supérieures (13; 22) et les têtes magnétiques inférieures (5; 25) sont pourvues d'une tête magnétique permanente et sont formées en joignant plus de deux groupes de blocs magnétiques permanents (15) les uns aux autres, et un espace est maintenu entre les groupes respectifs de blocs magnétiques permanents (15).

5. Appareil thérapeutique électromagnétique selon la revendication 3 ou 4, dans lequel l'intensité de champ magnétique statique entre les têtes magnétiques supérieures (13; 22) et les têtes magnétiques inférieures (5; 25) est de 0,5 T à 0,85 T (5000 G à 8500 G), la vitesse de rotation des têtes magnétiques inférieures (5; 25) est de 50 TPM à 600 TPM, et la distance entre les têtes magnétiques supérieures (13; 22) et les têtes magnétiques inférieures (5; 25) est de 200 mm à 400 mm, dans lequel les têtes magnétiques supérieures (13; 22) et les têtes magnétiques inférieures (5; 25) présentent toutes les deux une forme de disque, et le rapport de la distance entre des centres de têtes magnétiques respectives et le diamètre de la tête magnétique est de 1,2:1.

6. Appareil thérapeutique électromagnétique selon la revendication 5, dans lequel les disques magnétiques de tourmaline (11; 17) ou les blocs métalliques (23) sont distribués entre la toile de physiothérapie (12) et la doublure selon les points d'acuponcture d'un corps humain et sont pourvus de point convexes arqués (16) sur ceux-ci.

7. Appareil thérapeutique électromagnétique selon la revendication 6, dans lequel la toile de physiothérapie (12) est une structure du type fendu ou du type sac de couchage, la toile de physiothérapie (12) présentant la structure de type fendu comprenant au moins un manchon de tête présentant des ouvertures pour les oreilles, une ouverture pour le nez et une ouverture pour la bouche prévues dans celui-ci, un manchon de corps et des manchons de membres, dans lequel les bandes de pression de scellage étanche à l'eau sont prévues aux ouvertures du manchon de tête, du manchon de corps et du manchon de membres, et au manchon de tête, et dans lequel le manchon de corps et les manchons de membres sont connectés au réservoir de collecte de liquide de physiothérapie (8), respectivement.

8. Appareil thérapeutique électromagnétique selon la revendication 7, dans lequel plusieurs disques magnétiques de tourmaline (11; 17) ou pastilles de sel d'armoise (24) sont placée(e)s sur la surface supérieure de la couche de traitement (4).

9. Appareil thérapeutique électromagnétique selon la revendication 8, dans lequel la couche de traitement (4) est montée avec deux ou plus de deux rouleaux tournants en latex (10) qui sont montés en dessous des positions de la toile de physiothérapie (12) qui correspondent à la courbure du cou et/ou à la courbure de la taille et/ou à la courbure des jambes et/ou à la courbure de la cheville d'un corps humain; et chaque rouleau tournant en latex (10) est connecté à un micro-moteur qui entraîne les rouleaux tournants en latex (10) à tourner de façon indépendante.

10. Appareil thérapeutique électromagnétique selon la revendication 9, dans lequel des saillies radiales (19) sont prévues sur les rouleaux tournants en latex (10) et sont incorporées avec des particules de sel d'armoise (21) à la pointe, et un mandrin de chauffage métallique (20) est prévu à l'intérieur des rouleaux tournants en latex (10);
dans lequel l'alimentation électrique à haute fréquence (7) est apte à fournir un courant alternatif à haute fréquence à la bobine de chauffage par induction à haute fréquence (18), de telle sorte que le bloc métallique (23) et le mandrin de chauffage métallique (20) génèrent de la chaleur en même temps.

11. Appareil thérapeutique électromagnétique selon la revendication 10, dans lequel le cadre comprend une colonne (2), un plateau levant (1), un dispositif de levage et une plaque de montage de tête magnétique supérieure (14), dans lequel le plateau levant (1) est inséré dans la colonne (2), et le dispositif de levage est apte à entraîner le plateau levant (1) à exécuter un déplacement de montée et de descente; dans lequel le plateau levant (1) est connecté à la plaque de montage de tête magnétique supérieure (14), et les têtes magnétiques supérieures (13; 22) sont montées dans la plaque de montage de tête magnétique supérieure (14).

12. Appareil thérapeutique électromagnétique selon la revendication 11, dans lequel un dispositif de chauffage est monté à l'intérieur du réservoir de collecte de liquide de physiothérapie (8) et est connecté à un dispositif de commande de la température.

13. Appareil thérapeutique électromagnétique selon la revendication 12, dans lequel il comprend en outre un système de commande par ordinateur (3) qui est apte à se connecter aux et à commander les dispositifs d'entraînement rotatifs, la pompe de circulation (9), le micro-moteur, le dispositif de levage et le dispositif de commande de la température.

14. Appareil thérapeutique électromagnétique selon l'une quelconque des revendications précédentes, dans lequel le liquide de physiothérapie comprend un liquide de base et un ou plusieurs additifs herbacés de la médecine traditionnelle chinoise, dans lequel le liquide de base comprend le glycérol et l'huile d'olive, et le rapport de masse entre le glycérol et l'huile d'olive est de 1:1 à 1:3, dans lequel un ou plusieurs des additifs herbacés de la médecine traditionnelle chinoise est (sont) sélectionné (s) dans le groupe comprenant l'*Angelica sinensis,* le carthame, le *Notopterygium, l'Anredera cordifolia,* le *Taxus chinensis,* l'*Agrastache rugosa* et l'orpin filandreux ou une combinaison de ceux-ci; et le rapport de masse entre le liquide de base et ledit (lesdits) un ou plusieurs additif(s) herbacé(s) de la médecine traditionnelle chinoise est de 10:1 à 10:1,5.
